# EUROPÄISCHE PATENTSCHRIFT

(11) **EP 0 930 080 B1**
(45) Veröffentlichungstag und Bekanntmachung des Hinweises auf die Patenterteilung: **07.04.2004**
(21) Anmeldenummer: 99100400.3
(22) Anmeldetag: 18.01.1999
(51) Int. Cl.: A61M 1/16

(54) **Verfahren zur Steuerung einer Dialysevorrichtung mit einer Einrichtung zur Bereitstellung betriebsbereiter Dialysierflüssigkeit und Vorrichtung hierzu**
Method for controlling a dialysis apparatus with a means for preparation of dialysate and device for this
Méthode de contrôle d'un dispositif de dialyse avec un dispositif de préparation de dialysate et dispositif à cet effet

(30) Priorität: 19.01.1998 DE 19801768
(43) Veröffentlichungstag der Anmeldung: 21.07.1999
(73) Patentinhaber: Fresenius Medical Care Deutschland GmbH, 61352 Bad Homburg v.d.H. (DE)
(72) Erfinder: Müller, Carsten, 97502 Euerbach (DE)
(74) Vertreter: Luderschmidt, Schüler & Partner GbR

(56) Entgegenhaltungen:
- EP-A- 0 265 795
- EP-A- 0 694 312

## Beschreibung

Die Erfindung betrifft ein Verfahren zur Steuerung einer Vorrichtung zur extrakorporalen Blutbehandlung während der Unterbrechung der Blutbehandlung und eine Vorrichtung zur Bereitstellung betriebsbereiter Dialysierflüssigkeit.

Die Dialysierflüssigkeit wird üblicherweise on-line aus Frischwasser und einem Elektrolykonzentrat hergestellt, wobei letzteres eigensteril ist und Frischwasser üblicherweise keine Keime aufweist. Es ist jedoch nicht sichergestellt, daß die so hergestellte Dialysierflüssigkeit absolut steril ist. Die DE-A 36 41 843 beschreibt eine Hämodialysemaschine, in deren Dialysierflüssigkeitskreislauf stromauf der Dialyseflüssigkeit des Dialysators ein Sterilfilter geschaltet ist, um dem Dialysator eine absolut sterile Dialysierflüssigkeit bereitzustellen.

Bei der Hämodiafiltration kann die Dialysierflüssigkeit on-line aus Frischwasser und einem Elektrolytkonezentrat und die Substitutionsflüssigkeit on-line aus der Dialysierflüssigkeit hergestellt werden. Das Elektrolytkonzentrat ist zwar in der Regel steril und das Frischwasser weist üblicherweise keine Keime auf, es ist jedoch nicht sichergestellt, daß die on-line hergestellte Dialysierflüssigkeit absolut steril und pyrogenfrei ist, weshalb die Dialysierflüssigkeit zur Herstellung der Substitutionsflüssigkeit in den absolut sterilen und pyrogenfreien Zustand überführt wird. Dazu wird stromauf des Dialysators Dialysierflüssigkeit übernommen, welche durch mindestens einen Filter geleitet wird, der durch eine Keime zurückhaltende hydrophile Membran in zwei Kammern geteilt ist. Eine derartige Vorrichtung mit zwei im Dialysierflüssigkeitssystem angeordneten Sterilfiltern ist beispielsweise aus der DE-A 34 44 671 und der EP-A 0 692 268 bekannt.

Um ein Zusetzen der Sterilfilter mit Keimen oder Pyrogenen zu verhindern, ist es bekannt, die Membran des Sterilfilters zeitweise mit Dialysierflüssigkeit freizuspülen.

Die EP-A-0 694 312 beschreibt eine Hämodiafiltrationsvorrichtung mit einem im Dialysierflüssigkeitsweg angeordneten Sterilfilter, dessen Membran über eine Leitung mit Dialysierflüssigkeit freigespült werden kann. Eine Bypassleitung verbindet die zum Dialysator führende Dialysierflüssigkeitszuführleitung mit der vom Dialysator zum Auslauf führenden Dialysierflüssigkeitsabführleitung.

Bei den bekannten Blutbehandlungsvorrichtungen mit Sterilfiltern im Dialysierflüssigkeitsweg besteht die Gefahr, daß Dialysierflüssigkeit mit der falschen Temperatur oder Leitfähigkeit den Dialysator erreicht.

Während die erste Kammer des Sterilfilters beispielsweise mit Dialysierflüssigkeit durchspült wird, kommt es bei einer Unterbrechung der Behandlung zum Verweilen der Flüssigkeit in der zweiten Kammer des Filters. Nach Fortsetzung der Behandlung wird die dann ausgekühlte Flüssigkeit dem Dialysator zugeführt. Dialysierflüssigkeit mit zu hoher Temperatur kann den Dialysator dann erreichen, wenn eine Störung in der Temperaturregelung der Einrichtung zur Bereitstellung von Dialysierflüssigkeit vorliegt.

Der Erfindung liegt die Aufgabe zugrunde, ein Verfahren zur Steuerung einer Vorrichtung zur extrakorporalen Blutbehandlung während der Unterbrechung der Blutbehandlung anzugeben, das die Sicherheit der Blutbehandlung insofern erhöht, als den Dialysator auch nach dem Durchspülen des Sterilfilters Dialysierflüssigkeit mit einer vorgegebenen Temperatur oder Leitfähigkeit erreicht. Die Lösung dieser Aufgabe erfolgt erfindungsgemäß mit den im Patentanspruch 1 angegebenen Merkmalen.

Eine weitere Aufgabe der Erfindung liegt darin, eine Vorrichtung zur extrakorporalen Blutbehandlung zu schaffen, deren Sicherheit insofern erhöht ist, als den Dialysator auch nach dem Durchspülen des Sterilfilters Dialysierflüssigkeit mit einer vorgegebenen Temperatur oder Leitfähigkeit erreicht. Diese Aufgabe wird erfindungsgemäß mit den Merkmalen des Patentanspruchs 3 gelöst.

Bei dem erfindungsgemäßen Verfahren bzw. der erfindungsgemäßen Vorrichtung wird nach dem Schließen des ersten Bypassventils, wodurch der Spülvorgang der ersten Kammer des Sterilfilters unterbrochen wird, das zweite Bypassventil geöffnet, so daß die in der zweiten Kammer des Filters enthaltene Flüssigkeit, deren Temperatur und/oder Leitfähigkeit nicht einem vorgegebenen Wert entsprechen kann, über die zweite Bypassleitung in den Auslaß abgeführt wird. Dadurch wird vermieden, daß Dialysierflüssigkeit mit einer falschen Temperatur und/oder Zusammensetzung den Dialysator erreicht.

Während die Dialysierflüssigkeit unter Umgehung des Dialysators zu dem Auslaß strömt, wird die Temperatur und/oder Leitfähigkeit der abfließenden Dialysierflüssigkeit überwacht. Wenn die Abweichung der gemessenen Leitfähigkeit und/oder Temperatur von einem vorgegebenen Leitfähigkeits- und/oder Temperaturwert einen bestimmten Grenzwert unterschreitet, wird die zweite Bypassleitung wieder unterbrochen. Den Dialysator erreicht nunmehr Dialysierflüssigkeit mit der richtigen Temperatur und/oder Leitfähigkeit.

Wenn das zweite Bypassventil geöffnet ist, ist der Strömungsweg durch die erste Kammer des Dialysators unterbrochen. Die Unterbrechung des Strömungsweges erfolgt vorzugsweise mit einem stromauf des Dialysators in der Dialysierflüssigkeitszuführleitung angeordneten Sperrorgan.

Vorzugsweise ist ein weiteres Sperrorgan stromab des Dialysators in der Dialysierflüssigkeitsabführleitung vorgesehen.

Die Leitfähigkeit und/oder Temperatur der Dialysierflüssigkeit kann mit einer stromab des Sterilfilters in Strömungsrichtung vor dem Dialysator angeordneten ersten Meßeinrichtung oder einer in der Dialysierflüssigkeitsabführleitung stromab der zweiten Bypassleitung angeordneten zweiten Meßeinrichtung erfolgen, wenn die Dialysierflüssigkeit über die zweite Bypassleitung zu dem Auslauf strömt.

Um sicherzustellen, daß den Dialysator nur Dialysierflüssigkeit mit einer bestimmten Leitfähigkeit und/oder Temperatur erreicht, wird die Temperatur und/oder Leitfähigkeit der Dialysierflüssigkeit vorteilhafterweise mit einer stromauf des Sterilfilters in der Dialysierflüssigkeitszuführleitung angeordneten Meßeinrichtung überwacht. Wenn die Abweichung der gemessenen Temperatur und/oder Leitfähigkeit von einem vorgegebenen Temperatur- und/oder Leitfähigkeitswert einen bestimmten Grenzwert überschreitet, wird die erste Kammer des Sterilfilters über die erste Bypassleitung in den Durchfluß geschaltet und der zu dem Dialysator führende Teil des Dialysierflüssigkeitsweges wird unterbrochen. Die Leitfähigkeit und/oder Temperatur der Dialysierflüssigkeit wird nun mit der stromauf des Sterilfilters in der Dialysierflüssigkeitszuführleitung angeordneten Meßeinrichtung oder einer in der Dialysierflüssigkeitsabführleitung vorteilhafterweise stromab der ersten Bypassleitung angeordneten Meßeinrichtung überwacht. Wenn die Abweichung der Temperatur und/oder Leitfähigkeit von einem vorgegebenen Leitfähigkeits- und/oder Temperaturwert einen bestimmten Grenzwert unterschreitet, wird die Bypassleitung unterbrochen, so daß die Dialysierflüssigkeit wieder durch den Sterilfilter strömt. Dadurch wird sichergestellt, daß den Dialysator nur Dialysierflüssigkeit mit einer bestimmten Leitfähigkeit und/oder Temperatur erreicht.

Die Einhaltung genauer Temperatur- und/oder Leitfähigkeitswerte der Dialysierflüssigkeit ist insbesondere auch dann von Bedeutung, wenn aus der Dialysierflüssigkeit on-line Substituat gewonnen wird, das dem Patienten zugeführt wird.

Im folgenden werden unter Bezugnahme auf die Zeichnungen mehrere Ausführungsbeispiele einer Vorrichtung zur extrakorporalen Blutbehandlung mit einer Einrichtung zur Bereitstellung betriebsbereiter Dialysierflüssigkeit näher erläutert.

Es zeigen:
- Figur 1a:: eine Hämodialysevorrichtung mit einer Einrichtung zur Bereitstellung betriebsbereiter Dialysierflüssigkeit in schematischer Darstellung,
- Figur 1b:: eine schematische Darstellung einer zweiten Ausführungsform der Hämodialysevorrichtung und
- Figur 2:: eine Hämodiafiltrationsvorrichtung mit einer Einrichtung zur Bereitstellung betriebsbereiter Dialysierflüssigkeit in schematischer Darstellung.

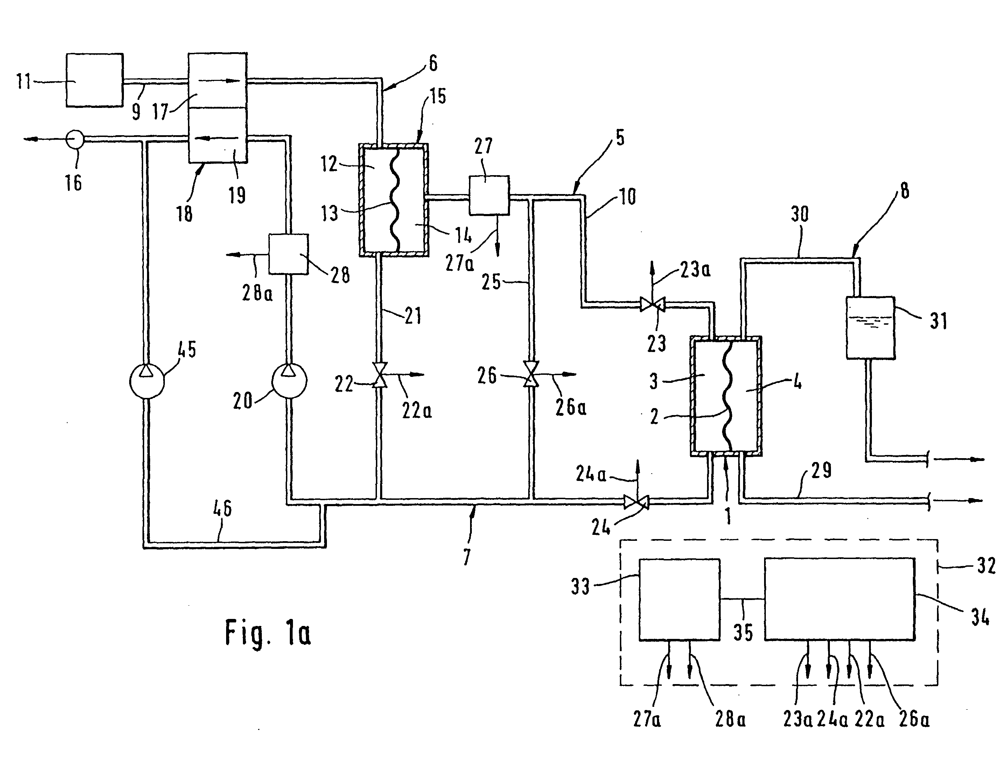

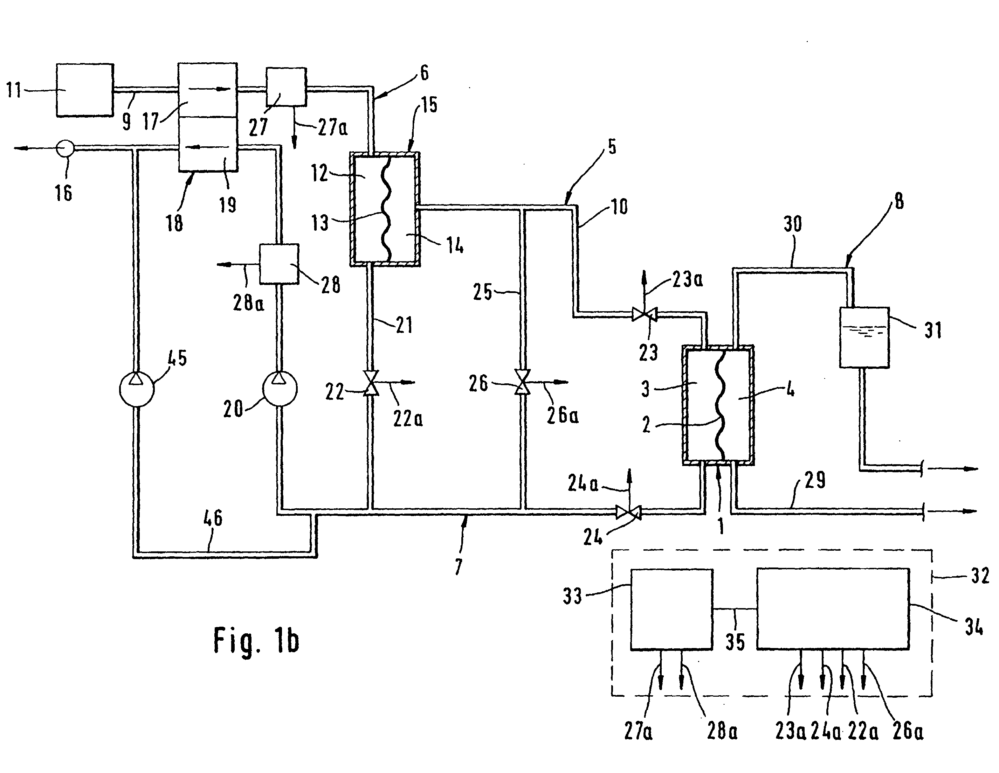

Figur 1 zeigt eine schematische Darstellung der wesentlichen Komponenten einer Hämodialysevorrichtung. Die Dialysevorrichtung weist einen Dialysator 1 auf, der durch eine semipermeable Membran 2 in eine von Dialysierflüssigkeit durchflossene erste Kammer 3 und eine von Blut durchflossene zweite Kammer 4 geteilt ist. Die erste Kammer 3 ist in einen Dialysierflüssigkeitsweg 5 geschaltet, der eine Dialysierflüssigkeitszuführleitung 6 und eine Dialysierflüssigkeitsabführleitung 7 aufweist, während die zweite Kammer 4 in einen Blutweg 8 geschaltet ist.

Die Dialysierflüssigkeitszuführleitung 6 des Dialysierflüssigkeitsweges 5 weist einen ersten Leitungsabschnitt 9 und einen zweiten Leitungsabschnitt 10 auf. Der erste Leitungsabschnitt 9 verbindet eine Einrichtung 11 zur Bereitstellung von Dialysierflüssigkeit mit dem Einlaß einer ersten Kammer 12 eines durch eine Keime zurückhaltende Membran 13 in die erste Kammer 12 und eine zweite Kammer 14 unterteilten Sterilfilters 15. Der zweite Zuleitungsabschnitt 10 verbindet den Auslaß der zweiten Kammer 14 des Sterilfilters 15 mit dem Einlaß der ersten Kammer 3 des Dialysators 1. Der Auslaß der ersten Kammer 3 des Dialysators 1 ist über die Dialysierflüssigkeitsabführleitung 7 mit einem Auslauf 16 verbunden.

In den ersten Leitungsabschnitt 9 der Dialysierflüssigkeitszuführleitung 6 ist die erste Bilanzierkammer 17 einer Bilanziereinrichtung 18 geschaltet, während die zweite Kammer 19 der Bilanziereinrichtung in die Dialysierflüssigkeitsabführleitung 7 geschaltet ist. Stromauf der zweiten Kammer 19 der Bilanziereinrichtung 18 ist in die Dialysierflüssigkeitsabführleitung 7 eine Dialysierflüssigkeitspumpe 20 geschaltet. Von dem Auslaß der ersten Kammer 12 des Sterilfilters 15 führt eine erste Bypassleitung 21, in die ein erstes Bypassventil 22 geschaltet ist, zu der Dialysierflüssigkeitsabführleitung 7 stromauf der Dialysierflüssigkeitspumpe 20.

Stromauf des Dialysators 1 ist in dem zweiten Leitungsabschnitt 10 der Dialysierflüssigkeitszuführleitung 6 ein erstes Absperrorgan 23 und stromab des Dialysators ist in der Dialysierflüssigkeitsabführleitung 7 ein zweites Absperrorgan 24 angeordnet. Eine zweite Bypassleitung 25, in die ein zweites Bypassventil 26 geschaltet ist, verbindet den zweiten Leitungsabschnitt 10 der Dialysierflüssigkeitszuführleitung 6 stromauf des ersten Absperrorgans 23 mit der Dialysierflüssigkeitsabführleitung 7 stromab des zweiten Absperrorgans 24.

In den zweiten Leitungsabschnitt 10 der Dialysierflüssigkeitszuführleitung 6 ist eine erste Einrichtung 27 zum Messen der Temperatur und/oder Leitfähigkeit der Dialysierflüssigkeit geschaltet, während in die Dialysierflüssigkeitsabführleitung 7 stromab der ersten Bypassleitung 21 eine zweite Einrichtung 28 zum Messen der Temperatur und/oder Leitfähigkeit der Dialysierflüssigkeit geschaltet ist.

Der Blutweg 8 weist eine vom Patienten kommende Blutzuführleitung 29 auf, die an den Eingang der zweiten Kammer 4 des Dialysators 1 angeschlossen ist. Der Ausgang der zweiten Kammer 4 des Dialysators führt über eine Blutabführleitung 30, in die eine Tropfkammer 31 geschaltet ist, zum Patienten.

Desweiteren weist die Hämodialysevorrichtung eine Ultrafiltrationsleitung 46 auf, in die eine Ultrafiltrationspumpe 45 geschaltet ist. Die Ultrafiltrationsleitung 46 zweigt von der Dialysierflüssigkeitsabführleitung 7 stromauf der Dialysierflüssigkeitspumpe 20 ab und mündet in die Dialysierflüssigkeitsabführleitung stromab der zweiten Kammer 19 der Bilanziereinrichtung 18.

Die Einrichtung 32 zur Bereitstellung betriebsbereiter Dialysierflüssigkeit weist eine Auswerteinrichtung 33 und eine Steuereinrichtung 34 auf. Die Auswerteinrichtung 33 empfängt die Meßwerte der ersten Meßeinrichtung 27 über eine Datenleitung 27a und die Meßwerte der zweiten Meßeinrichtung 28 über eine Datenleitung 28a. Die Steuereinrichtung 34 ist über eine Datenleitung 35 mit der Auswerteinrichtung 33 verbunden und steuert das erste und zweite Absperrorgan 23, 24 über die Steuerleitungen 23a, 24a und das erste und zweite Bypassventil 22, 26 über die Steuerleitungen 22a, 26a an.

Während der Dialysebehandlung sind das erste und zweite Absperrorgan 23, 24 geöffnet und das erste und zweite Bypassventil 22, 26 geschlossen, so daß Dialysierflüssigkeit aus der Einrichtung 11 zur Bereitstellung der Dialysierflüssigkeit über die erste Kammer 17 der Bilanziereinrichtung 18 und den Sterilfilter 15 in die erste Kammer 3 des Dialysators strömen kann. Aus der ersten Kammer des Dialysators strömt die Dialysierflüssigkeit dann über die zweite Kammer 19 der Bilanziereinrichtung 18 zu dem Auslauf 16.

Die Temperatur und/oder Leitfähigkeit der Dialysierflüssigkeit wird während der Dialysebehandlung laufend mit der ersten Meßeinrichtung 27 überwacht. In der Auswerteeinheit werden die Meßwerte der ersten Meßeinrichtung 27 mit einem vorgegebenen Temperatur- und/oder Leitfähigkeitswert verglichen. Wenn die Abweichung größer als ein bestimmter Grenzwert ist, öffnet die Steuereinrichtung 34 das erste Bypassventil 22 und schließt das erste und zweite Absperrorgan 23, 24. Prinzipiell ist es aber auch ausreichend, wenn nur ein Absperrorgan stromauf des Dialysators vorgesehen ist. Die Dialysierflüssigkeit strömt dann über die erste Bypassleitung 21 direkt zu dem Auslauf 16, wobei der Dialysator 1 von dem Dialysierflüssigkeitsweg abgetrennt ist. Die Temperatur und/oder Leitfähigkeit wird nun mit der zweiten Meßeinrichtung 28 überwacht. In der Auswerteinrichtung 33 werden die Meßwerte der zweiten Meßeinrichtung 28 mit einem vorgegebenen Temperatur- und/oder Leitfähigkeitswert verglichen. Wenn die Abweichung einen bestimmten Grenzwert unterschreitet, schließt die Steuereinrichtung 34 wieder das erste Bypassventil 22. Daraufhin können das erste und zweite Absperrorgan 23, 24 wieder geöffnet werden, so daß der Dialysator 1 wieder von Dialysierflüssigkeit durchströmt wird.

Um aber zu verhindern, daß nach dem Schließen des ersten Bypassventils 22 Dialysierflüssigkeit aus der zweiten Kammer 14 des Sterilfilters 15 in den Dialysator 1 strömt, deren Temperatur und/oder Leitfähigkeit nicht einem vorgegebenen Temperatur- und/oder Leitfähigkeitswert entspricht, wird der Dialysator nicht wieder sofort in den Dialysierflüssigkeitsweg geschaltet. Die Steuereinrichtung 34 öffnet nach dem Schließen des ersten Bypassventils 22 zunächst das zweite Bypassventil 26, wobei das erste und zweite Absperrorgan 23, 24 weiterhin geschlossen bleiben. Die in der zweiten Kammer 14 des Sterilfilters 15 befindliche Dialysierflüssigkeit kann nunmehr über die zweite Bypassleitung 25 unter Umgehung des Dialysators 1 zu dem Auslauf 16 abfließen. Die Temperatur und/oder Leitfähigkeit der Dialysierflüssigkeit wird dabei entweder mit der ersten oder der zweiten Meßeinrichtung 27, 28 überwacht. Erst wenn die Abweichung der Temperatur und/oder Leitfähigkeit von einem vorgegebenen Temperatur- und/oder Leitfähigkeitswert einen bestimmten Grenzwert unterschreitet, schließt die Steuereinrichtung 34 das zweite Bypassventil 26 und öffnet das erste und zweite Absperrorgan 23, 24. Dadurch wird erreicht, daß nur Dialysierflüssigkeit den Dialysator erreichen kann, deren Temperatur und/oder Leitfähigkeit einem vorgegebenen Temperatur- und/oder Leitfähigkeitswert entspricht.

Das Abführen der in der zweiten Kammer 14 des Sterilfilters 15 befindlichen Dialysierflüssigkeit über die zweite Bypassleitung 25 ist insbesondere dann von Vorteil, wenn die erste Kammer des Sterilfilters zuvor mit Dialysierflüssigkeit freigespült wurde, um ein Zusetzen der Membran mit Keimen oder Pyrogenen zu verhindern. Dadurch wird vermieden, daß die während des Freispülvorganges in der zweiten Kammer des Sterilfilters bereits abgekühlte Dialysierflüssigkeit den Dialysator erreicht.

Figur 1 b zeigt eine weitere Ausführungsform der Hämodialysevorrichtung. Diese unterscheidet sich von der unter Bezugnahme auf Figur 1 beschriebenen Ausführungsform dadurch, daß die erste Meßeinrichtung 27 nicht stromab des Sterilfilters 15 in dem zweiten Leitungsabschnitt 10 der Dialysierflüssigkeitszuführleitung 6, sondern in dem ersten Leitungsabschnitt 9 der Dialysierflüssigkeitszuführleitung 6 stromauf des Sterilfilters 15 angeordnet ist. Die Teile der Hämodialysevorrichtung gemäß Figur 1b, die denjenigen der Hämodialysevorrichtung gemäß Figur 1a entsprechen, sind mit denselben Bezugszeichen versehen. Bei dieser Ausführungsform erfolgt die Überwachung der Temperatur und/oder Leitfähigkeit der durch die erste Bypassleitung 21 strömenden Dialysierflüssigkeit entweder mit der ersten oder der zweiten Meßeinrichtung 27, 28. Die Überwachung der Temperatur und/oder Leitfähigkeit der durch die zweite Bypassleitung 25 strömenden Dialysierflüssigkeit kann bei dieser Ausführungsform allerdings nur mit der zweiten Meßeinrichtung 28 erfolgen. Ansonsten arbeitet die Hämodialysevorrichtung gemäß Figur 1b nach dem gleichen Programmablauf wie die Vorrichtung gemäß Figur 1 a, so daß sich eine weitere Erläuterung erübrigt.

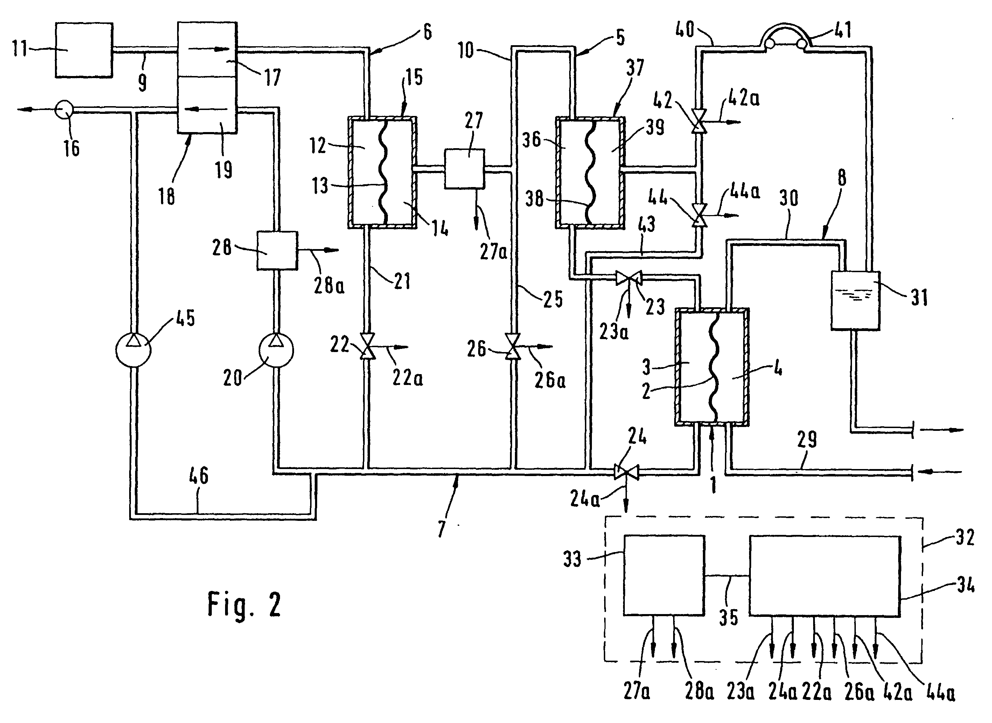

Figur 2 zeigt eine Hämodiafiltrationsvorrichtung in schematischer Darstellung. Die Hämodiafiltrationsvorrichtung unterscheidet sich von der unter Bezugnahme auf die Figuren 1a und 1b beschriebenen Hämodialysevorrichtung dadurch, daß in den zweiten Leitungsabschnitt 10 der Dialysierflüssigkeitszuführleitung 6 stromauf des ersten Absperrorgans 23 eine erste Kammer 36 eines zweiten Sterilfilters 37 geschaltet ist, der durch eine Keime zurückhaltende Membran 38 in die erste Kammer 36 und eine zweite Kammer 39 geteilt ist. Bei dem zweiten Sterilfilter 37 handelt es sich um einen Substituatfilter zur Gewinnung von Substituat aus der Dialysierflüssigkeit während der Hämodiafiltrationsbehandlung. Die zweite Kammer 39 des Substituatfilters 37 ist über eine Substituatleitung 40 mit der Tropfkammer 31 verbunden. In die Substituatleitung ist eine Substituatpumpe 41 geschaltet.

Stromauf der Substituatpumpe 41 befindet sich in der Substituatleitung 40 ein drittes Absperrorgan 42. Von der Substituatleitung 42 zweigt stromauf des dritten Absperrorgans 42 eine dritte Bypassleitung 43 ab, die stromab des zweiten Absperrorgans 24 in die Dialysierflüssigkeitsabführleitung 7 mündet. In die dritte Bypassleitung 43 ist ein drittes Bypassventil 44 geschaltet. Das dritte Absperrorgan 42 und das dritte Bypassventil 44 werden über weitere Steuerleitungen 42a, 44a von der Steuereinrichtung 34 angesteuert.

Während der Blutbehandlung kann dem Patienten über die Ultrafiltrationsleitung 46 mittels der Ultrafiltrationspumpe 45 Flüssigkeit entzogen werden. Flüssigkeit, die dem Patienten über die Pumpe 45 entzogen wird, wird gleichzeitig über die Substituatleitung 40 mittels der Substituatpumpe 41 wieder zugeführt, wobei die Substituatflüssigkeit on-line aus der Dialysierflüssigkeit gewonnen wird.

Die Überwachung der Temperatur und/oder Leitfähigkeit der Dialysierflüssigkeit erfolgt bei der Hämodiafiltrationsvorrichtung nach dem gleichen Programmablauf wie bei der Hämodialysevorrichtung gemäß der Figuren 1a und 1b. Insofern erübrigt sich eine weitere Erläuterung.

Die Hämodiafiltrationsvorrichtung hat nicht nur den Vorteil, daß den Dialysator immer Dialysierflüssigkeit mit der richtigen Temperatur und/oder Leitfähigkeit erreicht, sondern auch das aus der Dialysierflüssigkeit gewonnene Substituat immer die richtige Temperatur hat. Mit der ersten Bypassleitung 21 wird verhindert, daß Dialysierflüssigkeit mit der falschen Temperatur und/oder Leitfähigkeit den Dialysator bzw. Substituat mit der falschen Temperatur den Patienten erreicht. Desweiteren dient die erste Bypassleitung zum Freispülen des Dialysators. Die zweite Bypassleitung erlaubt es, die Parameter nach einer Unterbrechung z.B. nach dem Freispülen, wieder schnell auf die richtigen Werte einzustellen.

Zum Überprüfen des Substituatzweiges schließt die Steuereinrichtung 34 das dritte Absperrorgan 42 und öffnet das dritte Bypassventil 44, so daß das Substituat aus der zweiten Kammer 39 des Substituatfilters 37 zu dem Auslauf 16 abfließen kann. Sobald sich die richtigen Werte eingestellt haben, die mit der zweiten Meßeinrichtung 28 überwacht werden, schließt die Steuereinrichtung 34 wieder das dritte Bypassventil 44 und öffnet das dritte Absperrorgan 42, so daß dem Patienten wieder mit der Substituatpumpe 41 Substituat zugeführt werden kann.

## Patentansprüche

1. Verfahren zur Steuerung einer Vorrichtung zur extrakorporalen Blutbehandlung während der Unterbrechung der Blutbehandlung, wobei die Vorrichtung zur extrakorporalen Blutbehandlung aufweist:
- einen Dialysator, der durch eine semipermeable Membran in eine erste und eine zweite Kammer geteilt ist, wobei die erste Kammer in einen Dialysierflüssigkeitsweg und die zweite Kammer in einen Blutweg geschaltet ist,
- einen Sterilfilter, der durch eine Keime zurückhaltende Membran, in eine erste Kammer und eine zweite Kammer geteilt ist,
- eine Einrichtung zur Bereitstellung von frischer Dialysierflüssigkeit und einen Auslauf für verbrauchte Dialysierflüssigkeit,
- einen ersten Leitungsabschnitt einer Dialysierflüssigkeitszuführleitung, die von der Einrichtung zur Bereitstellung von frischer Dialysierflüssigkeit zu einem Einlaß der ersten Kammer des Sterilfilters führt,
- einen zweiten Leitungsabschnitt der Dialysierflüssigkeitszuführleitung, die von einem Auslaß der zweiten Kammer des Sterilfilters zu einem Einlaß der ersten Kammer des Dialysators führt,
- eine Dialysierflüssigkeitsabführleitung, die von einem Auslaß des Dialysators zu dem Auslauf führt,
- eine erste Bypassleitung, die von einem Auslaß der ersten Kammer des Sterilfilters zu der Dialysierflüssigkeitsabführleitung führt, wobei in die erste Bypassleitung ein erstes Bypassventil geschaltet ist,
- eine die Dialysierflüssigkeitszuführleitung mit der Dialysierflüssigkeitsabführleitung verbindende zweite Bypassleitung, in die ein zweites Bypassventil geschaltet ist und
- eine in dem ersten oder zweiten Leitungsabschnitt der Dialysierflüssigkeitszuführleitung angeordnete erste Einrichtung zur Messung der Temperatur und/oder Leitfähigkeit der Dialysierflüssigkeit und/oder eine in der Dialysierflüssigkeitsabführleitung stromab der ersten Bypassleitung angeordnete zweite Einrichtung zur Messung der Temperatur und/oder Leitfähigkeit der Dialysierflüssigkeit,
**gekennzeichnet durch** folgende Verfahrensschritte:
- Unterbrechen des Strömungsweges **durch** den Dialysator, wobei das zweite Bypassventil nach dem Schließen des ersten Bypassventils geöffnet, die Temperatur und/oder Leitfähigkeit mit der ersten oder zweiten Meßeinrichtung gemessen und die Meßwerte mit einem vorgegebenen Temperatur- und/oder Leitfähigkeitswert verglichen werden, und
- Schließen des zweiten Bypassventils, wenn die Abweichung von dem vorgegebenen Temperatur- und/oder Leitfähigkeitswert kleiner als ein bestimmter Grenzwert ist.

2. Verfahren nach Anspruch 1, **gekennzeichnet durch** folgende Verfahrensschritte:
- Messen der Temperatur und/oder Leitfähigkeit der Dialysierflüssigkeit mit der ersten Meßeinrichtung und Vergleichen der Meßwerte mit einem vorgegebenen Temperatur- und/oder Leitfähigkeitswert, während das erste und zweite Bypassventil geschlossen sind,
- Öffnen des ersten Bypassventils und Unterbrechen des Strömungsweges **durch** den Dialysator, wenn die Abweichung von dem vorgegebenen Temperatur und/oder Leitfähigkeitswert größer als ein bestimmter Grenzwert ist,
- Messen der Temperatur und/oder Leitfähigkeit der Dialysierflüssigkeit mit der ersten oder zweiten Meßeinrichtung und Vergleichen der Meßwerte mit einem vorgegebenen Temperatur- und/oder Leitfähigkeitswert und
- Schließen des ersten Bypassventils, wenn die Abweichung von dem vorgegebenen Wert kleiner als ein bestimmter Grenzwert ist.

3. Vorrichtung zur extrakorporalen Blutbehandlung mit
- einem Dialysator (1), der durch eine semipermeable Membran (2) in eine erste und eine zweite Kammer (3, 4) geteilt ist, wobei die erste Kammer in einen Dialysierflüssigkeitsweg (5) und die zweite Kammer in einen Blutweg (8) geschaltet ist,
- einem Sterilfilter (15), der durch eine Keime zurückhaltende Membran (13) in eine erste Kammer (12) und eine zweite Kammer (14) geteilt ist,
- einer Einrichtung (11) zur Bereitstellung von frischer Dialysierflüssigkeit und einem Auslauf (16) für verbrauchte Dialysierflüssigkeit,
- einem ersten Leitungsabschnitt (9) einer Dialysierflüssigkeitszuführleitung (6), die von der Einrichtung zur Bereitstellung von frischer Dialysierflüssigkeit zu einem Einlaß der ersten Kammer des Sterilfilters führt,
- einem zweiten Leitungsabschnitt (10) der Dialysierflüssigkeitszuführleitung (6), die von einem Auslaß der zweiten Kammer des Sterilfilters zu einem Einlaß der ersten Kammer des Dialysators führt,
- einer Dialysierflüssigkeitsabführleitung (7), die von einem Auslaß des Dialysators zu dem Auslauf führt,
- einer ersten Bypassleitung (21), die von einem Auslaß der ersten Kammer des Sterilfilters zu der Dialysierflüssigkeitsabführleitung führt, wobei in die erste Bypassleitung ein erstes Bypassventil (22) geschaltet ist und
- einer die Dialysierflüssigkeitszuführleitung mit der Dialysierflüssigkeitsabführleitung verbindende zweite Bypassleitung (25), in die ein zweites Bypassventil (26) geschaltet ist,
**gekennzeichnet durch**,
eine Einrichtung (32) zur Bereitstellung betriebsbereiter Dialysierflüssigkeit, die aufweist:
- eine in dem ersten oder zweiten Leitungsabschnitt der Dialysierflüssigkeitszuführleitung angeordnete erste Einrichtung (27) zur Messung der Temperatur und/oder Leitfähigkeit der Dialysierflüssigkeit und/oder eine in der Dialysierflüssigkeitsabführleitung stromab der ersten Bypassleitung angeordnete zweite Einrichtung (28) zur Messung der Temperatur und/oder Leitfähigkeit der Dialysierflüssigkeit,
- eine Auswerteinrichtung (33) zum Vergleichen der Meßwerte der ersten oder zweiten Meßeinrichtung mit vorgegebenen Leitfähigkeitswerten,
- eine Steuereinrichtung (34), über die das erste und zweite Bypassventil (22) ansteuerbar sind, wobei die Steuereinrichtung derart ausgebildet ist, daß nach dem Schließen des ersten Bypassventils (22) das zweite Bypassventil (26) geöffnet wird und das zweite Bypassventil (26) dann wieder geschlossen wird, wenn die Abweichung der Meßwerte der ersten oder zweiten Meßeinrichtung (27, 28) von einem vorgegebenen Temperatur- und/oder Leitfähigkeitswert kleiner als ein bestimmter Grenzwert ist.

4. Vorrichtung nach Anspruch 3, **dadurch gekennzeichnet, daß** ein erstes Absperrorgan (23), das in die Dialysierflüssigkeitszuführleitung (6) geschaltet ist, und ein zweites Absperrorgan (24), das in die Dialysierflüssigkeitsabführleitung (7) geschaltet ist, vorgesehen sind, wobei die Absperrorgane derart von der Steuereinrichtung (34) ansteuerbar sind, daß der Strömungsweg durch die erste Kammer (3) des Dialysators (1) unterbrochen ist, wenn das zweite Bypassventil (26) geöffnet ist.

5. Vorrichtung nach Anspruch 4, **dadurch gekennzeichnet, daß** die Steuereinrichtung (34) ferner derart ausgebildet ist, daß bei geschlossenem ersten und zweiten Bypassventil (22, 26) und geöffnetem ersten und zweiten Absperrorgan (23, 24) das erste Bypassventil (22) geöffnet und das erste oder das erste und zweite Absperrorgan (23, 24) geschlossen werden, wenn die Abweichung der Meßwerte der ersten Meßeinrichtung von einem vorgegebenen Temperatur- und/oder Leitfähigkeitswert größer als ein bestimmter Grenzwert ist, und daß das erste Bypassventil (22) dann wieder geschlossen wird, wenn die Abweichung der Meßwerte von der ersten oder zweiten Meßeinrichtung von einem vorgegebenen Temperatur- und/oder Leitfähigkeitswert kleiner als ein bestimmter Grenzwert ist.

## Claims

1. A method for controlling a device for extracorporeal blood treatment during the interruption of the blood treatment, whereby the device for extracorporeal blood treatment has:
- a dialyser, which is split by a semi-permeable membrane into a first and a second chamber, whereby the first chamber is connected into a dialysis fluid path and the second chamber into a blood path,
- a sterile filter, which is split by a germ-restraining membrane into a first chamber and a second chamber,
- a device for the preparation of fresh dialysis fluid and a drain for used dialysis fluid,
- a first pipe section of a dialysis fluid feed pipe, which leads from the device for the preparation of fresh dialysis fluid to an inlet of a first chamber of the sterile filter,
- a second pipe section of the dialysis fluid feed pipe, which leads from an outlet of the second chamber of the sterile filter to an inlet of the first chamber of the dialyser,
- a dialysis fluid drainage pipe, which leads from an outlet of the dialyser to the drain,
- a first bypass pipe, which leads from an outlet of the first chamber of the sterile filter to the dialysis fluid drainage pipe, whereby a first bypass valve is connected into the first bypass pipe,
- a second bypass pipe connecting the dialysis fluid feed pipe with the dialysis fluid drainage pipe, into which second bypass pipe there is connected a second bypass valve and
- a first device, arranged in the first or second pipe section of the dialysis fluid feed pipe, for measuring the temperature and/or conductivity of the dialysis fluid and/or a second device, arranged in the dialysis fluid drainage pipe downstream of the first bypass pipe, for measuring the temperature and/or conductivity of the dialysis fluid,
**characterised by** the following process steps:
- interruption of the flow path through the dialyser, whereby the second bypass valve is opened after the first bypass valve is closed, the temperature and/or conductivity is measured with the first or second measuring device and the measurement values are compared with a predetermined temperature and/or conductivity value, and
- closure of the second bypass valve when the deviation from the predetermined temperature and/or conductivity value is smaller than a specified limiting value.

2. The method according to claim 1,**characterised by** the following process steps:
- measurement of the temperature and/or conductivity of the dialysis fluid with a first measuring device and comparison of the measurement values with a predetermined temperature and/or conductivity value, while the first and second bypass valves are closed,
- opening of the first bypass valve and interruption of the flow path through the dialyser when the deviation from the predetermined temperature and/or conductivity value is greater than a specified limiting value,
- measurement of the temperature and/or conductivity of the dialysis fluid with the first or second measuring device and comparison of the measurement values with a predetermined temperature and/or conductivity value and
- closure of a first bypass valve when the deviation from the predetermined value is smaller than a specified limiting value.

3. A device for extracorporeal blood treatment with
- a dialyser (1), which is split by a semi-permeable membrane (2) into a first and a second chamber (3,4), whereby the first chamber is connected into a dialysis fluid path (5) and the second chamber into a blood path (8),
- a sterile filter (15), which is split by a germ-restraining membrane (13) into a first chamber (12) and a second chamber (14),
- a device (11) for the preparation of fresh dialysis fluid and a drain (16) for used dialysis fluid,
- a first pipe section (9) of a dialysis fluid feed pipe (6), which leads from the device for the preparation of fresh dialysis fluid to an inlet of the first chamber of the sterile filter,
- a second pipe section (10) of the dialysis fluid feed pipe (6), which leads from an outlet of the second chamber of the sterile filter to an inlet of the first chamber of the dialyser,
- a dialysis fluid drainage pipe (7), which leads from an outlet of the dialyser to the drain,
- a first bypass pipe (21), which leads from an outlet of the first chamber of the sterile filter to the dialysis fluid drainage pipe, whereby a first bypass valve (22) is connected into the first bypass pipe,
- a second bypass pipe (25) connecting the dialysis fluid feed pipe with the dialysis fluid drainage pipe, into which second bypass pipe there is connected a second bypass valve (26),
**characterised by**
a device (32) for the preparation of dialysis fluid ready for use, which has:
- a first device (27), arranged in the first or second pipe section of the dialysis fluid feed pipe, for measuring the temperature and/or conductivity of the dialysis fluid and/or a second device (28), arranged in the dialysis fluid drainage pipe downstream of the first bypass pipe, for measuring the temperature and/or conductivity of the dialysis fluid,
- an analysing unit (33) for comparing the measurement values of the first or second measuring device with predetermined conductivity values,
- a control device (34), by means of which the first and second bypass valves (22) can be triggered, whereby the control device is designed in such a way that, after closure of the first bypass valve (22), the second bypass valve (26) is opened and the second bypass valve (26) is then closed again when the deviation of the measurement of values of the first or second measuring device (27, 28) from the predetermined temperature and/or conductivity value is less than a specified limiting value.

4. The device according to claim 3, **characterised in that** a first shut-off device (23), which is connected into the dialysis fluid feed pipe (6), and a second shut-off device (24), which is connected into the dialysis fluid drainage pipe (7), are provided, whereby the shut-off devices can be triggered by the control device (34) in such a way that the flow path through the first chamber (3) of the dialyser (1) is interrupted when the second bypass valve (36) is opened.

5. The device according to claim 4, **characterised in that** the control device (34) is further designed in such a way that, when the first and second bypass valves (22, 26) are closed and the first and second shut-off devices (23, 24) are opened, the first bypass valve (22) is opened and the first or the second and first shut-off devices (23, 24) are closed when the deviation of the measurement values of the first measuring device from a predetermined temperature and/or conductivity value is greater than a specified limiting value, and that the first bypass valve (22) is then closed again when the deviation of the measurement values of the first or second measuring device from a predetermined temperature and/or conductivity value is smaller than a specified limiting value.

## Revendications

1. Procédé pour commander un dispositif pour le traitement extracorporel du sang pendant l'interruption du traitement du sang, où le dispositif pour le traitement extracorporel du sang comprend :
- un dialyseur qui est divisé par une membrane semiperméable en une première et une seconde chambre, où la première chambre est branchée dans une voie de liquide de dialyse et la seconde chambre est branchée dans une voie de sang,
- un filtre stérile qui est divisé par une membrane retenant les germes en une première chambre et une seconde chambre,
- un dispositif pour préparer du liquide de dialyse frais et une évacuation pour le liquide de dialyse usé,
- une première section de conduit d'un conduit d'apport de liquide de dialyse qui mène du dispositif pour préparer du liquide de dialyse frais à une entrée de la première chambre du filtre stérile,
- une seconde section de conduit du conduit d'apport de liquide de dialyse qui mène d'une sortie de la seconde chambre du filtre stérile à une entrée de la première chambre du dialyseur,
- un conduit d'évacuation de liquide de dialyse qui mène d'une sortie du dialyseur à l'évacuation,
- un premier conduit de dérivation qui mène d'une sortie de la première chambre du filtre stérile au conduit d'évacuation de liquide de dialyse,
où une première vanne de dérivation est branchée dans le premier conduit de dérivation,
- un second conduit de dérivation reliant le conduit d'apport de liquide de dialyse au conduit d'évacuation de liquide de dialyse, dans lequel est branchée une seconde vanne de dérivation et
- un premier dispositif, disposé dans la première ou seconde section de conduit du conduit d'apport de liquide de dialyse, pour mesurer la température et/ou la conductibilité du liquide de dialyse et/ou un second dispositif, disposé dans le conduit d'évacuation de liquide de dialyse en aval du premier conduit de dérivation, pour mesurer la température et/ou la conductibilité du liquide de dialyse,
**caractérisé par** les étapes de procédé suivantes :
- interruption de la voie d'écoulement dans le dialyseur, où la seconde vanne de dérivation est ouverte après la fermeture de la première vanne de dérivation, la température et/ou la conductibilité sont mesurées avec le premier ou le second dispositif de mesure et les valeurs mesurées sont comparées avec une valeur de température et/ou de conductibilité prédéterminée, et
- fermeture de la seconde vanne de dérivation quand l'écart par rapport à la température et/ou la conductibilité prédéterminées est inférieur à une valeur limite déterminée.

2. Procédé selon la revendication 1, **caractérisé par** les étapes de procédé suivantes :
- mesure de la température et/ou de la conductibilité du liquide de dialyse avec le premier dispositif de mesure et comparaison des valeurs mesurées avec une valeur de température et/ou de conductibilité prédéterminée, pendant que la première et seconde vannes de dérivation sont fermées,
- ouverture de la première vanne de dérivation et interruption de la voie d'écoulement dans le dialyseur quand l'écart par rapport à la valeur de température et/ou de conductibilité prédéterminée est supérieur à une valeur limite déterminée,
- mesure de la température et/ou de la conductibilité du liquide de dialyse avec le premier ou second dispositif de mesure et comparaison des valeurs mesurées avec une valeur de température et/ou de conductibilité prédéterminée, et
- fermeture de la première vanne de dérivation quand l'écart par rapport à la valeur prédéterminée est inférieur à une valeur limite déterminée.

3. Dispositif pour le traitement extracorporel du sang comportant :
- un dialyseur (1) qui est divisé par une membrane semiperméable (2) en une première et une seconde chambre (3, 4), où la première chambre est branchée dans une voie de liquide de dialyse (5) et la seconde chambre est branchée dans une voie de sang (8),
- un filtre stérile (15) qui est divisé par une membrane retenant les germes (13) en une première chambre (12) et une seconde chambre (14),
- un dispositif (11) pour préparer du liquide de dialyse frais et une évacuation (16) pour le liquide de dialyse usé,
- une première section de conduit (9) d'un conduit d'apport de liquide de dialyse (6) qui mène du dispositif pour préparer du liquide de dialyse frais à une entrée de la première chambre du filtre stérile,
- une seconde section de conduit (10) du conduit d'apport de liquide de dialyse (6) qui mène d'une sortie de la seconde chambre du filtre stérile à une entrée de la première chambre du dialyseur,
- un conduit d'évacuation de liquide de dialyse (7) qui mène d'une sortie du dialyseur à l'évacuation,
- un premier conduit de dérivation (21) qui mène d'une sortie de la première chambre du filtre stérile au conduit d'évacuation de liquide de dialyse, où
- une première vanne de dérivation (22) est branchée dans le premier conduit de dérivation et
- un second conduit de dérivation (25), reliant le conduit d'apport de liquide de dialyse au conduit d'évacuation de liquide de dialyse, dans lequel est branchée une seconde vanne de dérivation (26),
**caractérisé par**
un dispositif (32) pour préparer du liquide de dialyse prêt à l'emploi, qui comprend :
- un premier dispositif (27), disposé dans la première ou seconde section de conduit du conduit d'apport de liquide de dialyse, pour mesurer la température et/ou la conductibilité du liquide de dialyse et/ou un second dispositif (28), disposé dans le conduit d'évacuation de liquide de dialyse en aval du premier conduit de dérivation, pour mesurer la température et/ou la conductibilité du liquide de dialyse,
- un dispositif d'évaluation (33) pour comparer les valeurs mesurées du premier ou second dispositif de mesure avec des valeurs de conductibilité prédéterminées,
- un dispositif de commande (34) par le biais duquel la première et la seconde vanne de dérivation (22) peuvent être commandées, où le dispositif de commande est agencé de telle manière qu'après la fermeture de la première vanne de dérivation (22), la seconde vanne de dérivation (26) est ouverte, après quoi la seconde vanne de dérivation (26) est fermée de nouveau quand l'écart des valeurs mesurées du premier ou second dispositif de mesure (27, 28) par rapport à une valeur de température et/ou de conductibilité. prédéterminée est inférieur à une valeur limite déterminée.

4. Dispositif selon la revendication 3, **caractérisé en ce qu'**il est prévu un premier organe d'arrêt (23), qui est branché dans le conduit d'apport de liquide de dialyse (6), et un second organe d'arrêt (24), qui est branché dans le conduit d'évacuation de liquide de dialyse (7), où les organes d'arrêt peuvent être commandés par le dispositif de commande (34) de telle manière que la voie d'écoulement dans la première chambre (3) du dialyseur (1) est interrompue quand la seconde vanne de dérivation (26) est ouverte.

5. Dispositif selon la revendication 4, **caractérisé en ce que** le dispositif de commande (34) est en outre agencé de telle manière que, lorsque la première et la seconde vanne de dérivation (22, 26) sont fermées et lorsque le premier et le second organe d'arrêt (23, 24) sont ouverts, la première vanne de dérivation (22) est ouverte et le premier ou le premier et le second organe d'arrêt (23, 24) sont fermés quand l'écart des valeurs mesurées du premier dispositif de mesure par rapport à une valeur de température et/ou de conductibilité prédéterminée est supérieur à une valeur limite déterminée, et **en ce que** la première vanne de dérivation (22) est ensuite fermée de nouveau quand l'écart des valeurs mesurées par le premier ou le second dispositif de mesure par rapport à une valeur de température et/ou de conductibilité prédéterminée est inférieur à une valeur limite déterminée.
